# EUROPÄISCHE PATENTSCHRIFT

(11) **EP 2 268 788 B1**
(45) Veröffentlichungstag und Bekanntmachung des Hinweises auf die Patenterteilung: **20.07.2016**
(21) Anmeldenummer: 09721306.0
(22) Anmeldetag: 13.03.2009
(51) Int. Cl.: C12M 1/00, C12M 1/12, C12M 3/06, C12M 1/34

(54) **BIOREAKTOR**
BIOREACTOR
BIORÉACTEUR

(30) Priorität: 20.03.2008 DE 102008015386
(43) Veröffentlichungstag der Anmeldung: 05.01.2011
(73) Patentinhaber: Sartorius Stedim Biotech GmbH, 37079 Göttingen (DE)
(72) Erfinder: WÜNN, Eberhard, 37077 Göttingen (DE); GRELLER, Gerhard, 37085 Göttingen (DE); ADAMS, Thorsten, 37085 Göttingen (DE)
(86) Internationale Anmeldenummer: PCT/EP2009/001828
(87) Internationale Veröffentlichungsnummer: WO 2009/115241

(56) Entgegenhaltungen:
- EP-A- 0 086 539
- WO-A-86/07605
- WO-A-2007/038893
- DE-A1- 19 817 081
- US-A1- 2004 159 616
- US-B2- 6 544 788

## Beschreibung

Die Erfindung bezieht sich auf einen Bioreaktor zur Kultivierung von Mikroorganismen und Zellen.

Um Mikroorganismen oder Zellen tierischer oder pflanzlicher Herkunft in einem Bioreaktor in hoher Dichte zu kultivieren ist es oft notwendig die Konzentration toxischer Stoffwechselprodukte wie Ammonium oder Lactat im Kulturmedium so gering wie möglich zu halten. Dies wird erreicht, indem kontinuierlich zellfreies belastetes Medium aus dem Bioreaktor entfernt und durch frisches Kulturmedium ersetzt wird. Die Separation von Zellen und Kulturmedium kann dabei beispielsweise durch Zentrifugation oder Filtration erreicht werden. Der Vorgang des Ausschleusens und volumengleichen Ersetzens von Kulturmedium wird als Perfusion bezeichnet. Der Perfusionsschritt kann außerhalb des Bioreaktors erfolgen, was jedoch apparativ aufwändig und mit einer hohen Kontaminationsgefahr verbunden ist. Bei Bioreaktoren mit flexiblen Wänden kann die Entnahme von zellfreiem Kulturmedium auf sehr einfache Art erfolgen, indem ein Perfusionsfilter an die Innenwand des Reaktors angebracht wird.

Ein derartiger Einweg-Perfusionsbioreaktor wird von der Sartorius Stedim Biotech GmbH unter dem Namen CultiBag^{®} RM vertrieben. Das Durchmischen erfolgt hier mittels einer Kippvorrichtung, welche das Kulturmedium im flachen Beutel beständig hin- und herlaufen lässt. Das Abtrennen des zellfreien Kulturmediums erfolgt über ein Filtrationsmodul in Form einer Filtertasche, bestehend aus einem großporigen Filtermedium, dessen Peripherie mit einer der inneren Bioreaktorwände verschweißt ist.

Die Notwendigkeit, ein für Zellen undurchlässiges, für sonstige Inhaltsstoffe der Zellsuspension gut durchlässiges Filtermedium zu wählen, begrenzt die effektive Porengröße der Filterschicht bei maximal 10 µm. Geeignete Filter sind Mikrofiltrationsmembranen, die bekanntermaßen bei großem Leerraumvolumen eine enge Porengrößenverteilung aufweisen. Ein Nachteil der bisher als Perfusionsmembran in den Beuteln verwendeten Materialien ist ihre für den Dauereinsatz unzureichende mechanische Festigkeit.

Nachteile dieses Bioreaktors ergeben ferner sich aus dem Umstand, dass es bei dem Filtermedium zu Verwerfungen und Faltenbildungen des Filtermediums kommt, was bei längerem Betrieb zum Brechen des Filtermediums aufgrund der mechanischen Beanspruchung in den Faltenbereichen kommt, die durch die Bewegung des Kulturmediums hervorgerufen wird.

Aus US-Patent 6,544,788 B2 ist ein Bioreaktor mit flexiblen Wänden bekannt, der zum Zweck der Perfusion über ein auf der Oberfläche des Kulturmediums aufschwimmendes Filtermodul verfügt. Der mechanisch stabile Schwimmkörper ist allerdings von begrenztem Nutzen, wenn in der Startphase einer Zellkultur mit reduziertem Kulturvolumen gearbeitet wird. Dann kann das Schwimmergehäuse auf die Beutelwand schlagen und dieselbe beschädigen. Ferner sind die für die Perfusion benötigten Filterflächen so groß, dass der Austausch von Gasen mit dem Kulturmedium, insbesondere von Sauerstoff beeinträchtigt wird.

Die Aufgabe der vorliegenden Erfindung besteht deshalb darin, einen Bioreaktor mit einem zur Perfusion geeigneten Filtermodul vorzuschlagen, das sich durch eine hohe mechanische Stabilität im Dauergebrauch auszeichnet und einen Austausch von Gasen mit dem Kulturmedium nicht behindert.

Die Aufgabe wird gelöst durch einen Bioreaktor mit mindestens einer Filtertasche, die an der flüssigkeitsbenetzten Innenfläche einer Wand des Bioreaktors fixiert ist und in ihrem Inneren über einen Sammel- und/oder Verteilerraum verfügt, der mit mindestens einem Anschluss für ein Zu- und/oder Abführen von Medien kommunizierend verbunden ist, wobei wenigstens eine Filtertaschenwand zumindest teilweise durch ein hydrophiles Filtermedium gebildet ist. Überraschenderweise wurde gefunden, dass selbst bei einer Filtertasche, welche eine gesamte Wand des Bioreaktors bedeckt, eine Faltenbildung des Filtermediums vermieden wird, wenn das Filtermedium in Wasser einen Quellungs- und/oder Schrumpfungsgrad von höchstens 1% aufweist.

Der Quellungsgrad des Filtermediums wird dabei wie folgt bestimmt:
Aus einem Abschnitt eines Filtermediums, welches bei Raumtemperatur in Umgebungsluft auf Gewichtskonstanz getrocknet wurde, wird ein kreisförmiges Stück mit definiertem Durchmesser ausgestanzt, gekennzeichnet und bei RT in RO-Wasser inkubiert (RO = Reverse Osmose oder Umkehrosmose). Nach 24 h wird die Probe faltenfrei auf einer porenfreien, glatten Oberfläche abgelegt und der Durchmesser in Bahnrichtung und quer zur Bahnrichtung bestimmt. Aus dem Verhältnis des Durchmessers der in Wasser gelagerten Probe zum ursprünglichen Durchmesser errechnet man den prozentualen Quellungsgrad. Der Schrumpfungsgrad wird analog bestimmt, indem eine in RO-Wasser inkubierte Probe vermessen wird. Nach 24 h Trocknen bis zur Gewichtskonstanz bei RT, wird der Durchmesser erneut vermessen und der Schrumpfungsgrad errechnet.

Dadurch, dass die Filtertasche an der Innenfläche einer Wand des Bioreaktors fixiert ist und nicht auf der Oberfläche des Kulturmediums aufschwimmt, wird der Gasaustausch zwischen Gasphase und Kulturmedium nicht beeinträchtigt. Durch den im Bioreaktor herrschenden Überdruck oder durch Anlegen eines Unterdruckes in der Filtertasche wird das Kulturmedium über das hydrophile Filtermedium in die Filtertasche befördert, wobei der Sammel- und/oder Verteilerraum im Innern der Filtertasche als Sammelraum für das filtrierte Kulturmedium wirkt, welches über einen Anschluss aus dem Bioreaktor abführbar ist.

Unter hydrophilen Filtermedien sollen solche verstanden werden, bei denen ein RO-Wassertropfen von 10 µl, der druckfrei auf ein getrocknetes Filtermedium aufgetragen wird, innerhalb von 600 Sekunden bei RT in ruhiger Umgebungsluft vollständig von der porösen Matrix des Filtermediums aufgenommenen wird.

Bevorzugt ist der Bioreaktor so ausgeführt, dass die Wand des Bioreaktors eine der Filtertaschenwände bildet. In weiteren Ausführungen ist die Filtertasche über Distanzhalter an der Innenwand des Bioreaktors fixiert, oder es sind mehrere Filtertaschen als Filtertaschenstapel ausgebildet, die durch zwischen den Filtertaschen liegende offene Kanäle getrennt sind. Durch beide Maßnahmen wird die zur Filtration zur Verfügung stehende Filterfläche weiter vergrößert, was zu einer erheblichen Verbesserung der Perfusion beiträgt, indem die Verblockung des Filtermediums vermieden wird.

In einer weiteren bevorzugten Ausführungsform der Erfindung besteht wenigstens eine Filtertaschenwand zumindest teilweise aus einem hydrophoben Filtermedium. Der Kopfraum über dem Kulturmedium ist mit Gas beaufschlagbar. So kann zum Beispiel während einer Unterbrechung des der Filtration über eine zumindest teilweise hydrophobe Filtertasche Gas in den Bioreaktor eingeleitet werden. Dabei wirkt der Sammel- und/oder Verteilerraum der Filtertasche als Verteilerraum für das Gas. Als Gas wird vorzugsweise Stickstoff, Luft oder Sauerstoff eingeleitet. Der Sammel- und/oder Verteilerraum der Filtertasche kann auch fluiddicht unterteilt sein, derart, dass das hydrophile Filtermedium an den Sammel- und das hydrophobe Filtermedium an den Verteilerraum angrenzt. Bei dieser Ausführungsform ist über die Filtertasche gleichzeitig Kulturmedium abführbar und Gas zuführbar, wobei jeweils zwei separate Anschlüsse für das Zu- und Abführen der Medien vorhanden sind.

Das hydrophile Filtermedium besteht aus einem Membranfilter (weiter als Membran bezeichnet), einer aus Fasern aufgebauten Filterschicht (Gewebe, Vliesstoff) oder einer aus Partikeln gesinterten Schicht. Derartige hydrophile Filtermedien bestehen aus natürlichen oder synthetischen Polymeren sowie aus anorganischen Werkstoffen, wobei die Hydrophilie durch Maßnahmen der Modifizierung der Polymere oder Filtermedien, die dem Fachmann geläufig sind, erzeugt oder verstärkt werden kann. Bevorzugte polymere Materialien für die Herstellung hydrophiler Filtermedien sind aliphatische Polyamide, Polysulfone und Polyethersulfone, Polyester, Polyvinylidenhalogenide, Acrylpolymere, Acryl-Copolymere und Zelluloseester.

Hydrophobe Filtermedien bestehen aus natürlichen oder synthetischen Polymeren sowie aus anorganischen Werkstoffen, wobei die Hydrophobizität durch Maßnahmen der Modifizierung der Polymere oder Filtermedien, die dem Fachmann geläufig sind, erzeugt oder verstärkt werden kann. Bevorzugte polymere Materialien für die Herstellung hydrophober Filtermedien sind Polyalkene, halogenierte Polyalkene, Polysulfone und Polyethersulfone, aliphatische Polyamide, Polyester, Acrylpolymere, Acryl-Copolymere und Zelluloseester.

In einer bevorzugten Ausführungsform der Erfindung ist das hydrophile und/oder hydrophobe Filtermedium mikroporös. Unter mikroporös wird ein Porengrößenbereich von 0,1 bis 20 µm verstanden. Das hydrophile Filtermedium wird bevorzugt in einer Porengröße zwischen 0,1 bis 10 µm und das hydrophobe Filtermedium wird bevorzugt in einer Porengröße innerhalb eines Bereiches von 0,1 bis 0,8 µm eingesetzt.

In einer weiteren bevorzugten Ausführungsform der Erfindung besteht das Filtermedium aus einer Membran, die auf mindestens der Seite, die dem Sammel- und/oder Verteilerraum der Filtertasche zugewandt ist, mit einem porösen Flächengebilde aus thermoplastischen Polymerfasern, die bei Kontakt mit dem wässrigen Kulturmedium nicht oder nur minimal quellen, verbunden ist. Das poröse Flächengebilde besteht aus einem Vlies, Gewebe, Gewirke oder Gitter, wobei ein Vlies aus Polymerfasern bevorzugt ist. Solcherart mechanisch verstärkte, mikroporöse, hydrophile oder hydrophobe Membranen weisen bei Kontakt mit dem Kulturmedium keine oder nur minimale Quellung (< 1%) auf und eignen sich daher besonders als Filtermedium.

Die Verbindung ist durch Laminieren der Membran auf das Flächengebilde unter Einwirkung von Druck und erhöhter Temperatur herstellbar oder als integrale Verbindung ausgeführt, bei der das Membranmaterial teilweise oder vollständig in das Flächengebildes eingedrungen ist. Letzteres wird während des Membranherstellungsprozesses bewirkt.
So weist eine unverstärkte hydrophile Polyamidmembran (PA6.6) bei Benetzung mit Wasser eine Quellung von ca. 2% auf. Wird eine PA6.6-Membran mit einem 12 g /m² Polyestervlies verstärkt, so beträgt die Quellung bei Benetzung mit Wasser nur 0,4%. Eine mikroporöse PA6.6-Membran, die mit einem Polyethylen-/Polypropylenvlies einseitig durch Laminieren verstärkt ist, weist in Wasser nur eine Dimensionsänderung von kleiner 0,35 % auf. Daraus gefertigte Filtertaschen wiesen selbst nach 7-tägigem Gebrauch in einem Bioreaktor keinerlei Verwerfungen bzw. Faltenbildungen auf.

Vorzugsweise sind die thermoplastischen Polymerfasern hydrophob. Durch die hydrophoben Eigenschaften stellt das poröse Flächengebilde eine erste hydrophobe Barriere für das Kulturmedium dar. Diese erste hydrophobe Barriere ist vorzugsweise erst ab einer Druckdifferenz von 20 mbar für das Kulturmedium zur Perfusion überwindbar. Durch die hydrophobe Barriere wird verhindert, dass Kulturmedium beispielsweise während der Anzucht- oder Startphase in das Innere der Filtertasche eindringt und als Kulturmedium während dieser Zeit und zu Zeiten, in denen Kulturmedium nicht abgeführt werden soll, dem Bioreaktor nicht mehr zur Verfügung steht.

Zur Erhöhung der hydrophoben Wirkung kann der Fachmann auf geläufige Maßnahmen der Modifizierung zurückgreifen. Auch ist die Wahl eines aus dünneren Fasern hergestellten, verdichteten Vliesmaterials als ein poröses Flächengebilde möglich.

Darüber hinaus hat es sich besonders bewährt, die Membran auf beiden Seiten mit einem porösen Flächengebilde aus thermoplastischen Polymerfasern zu verbinden, beispielsweise weist eine Polyethersulfonmembran, die durch ein Polyethylen- /Polypropylenvlies zweiseitig verstärkt wird, in Wasser einen Quellungsgrad von nur 0 - 0,2 % auf und ist für die Verwendung als Filtertaschenwand in Bioreaktoren besonders gut geeignet.

Das poröse Flächengebilde, das nicht an den Sammel- und/oder Verteilerraum der Filtertasche angrenzt, stellt dabei im Falle der Verwendung hydrophober Polymerfasern nicht nur eine zweite hydrophobe Barriere für das Kulturmedium dar, sondern es bildet auch einen wirksamen mechanischen Oberflächenschutz für die Membran. Diese zweite hydrophobe Barriere ist für das Kulturmedium vorzugsweise ab einer Druckdifferenz von 1 mbar überwindbar gestaltet. Die beidseitige Verstärkung des mikroporösen Membranfilters verbessert darüber hinaus auch die Planlageeigenschaften des laminierten Filtermediums und erleichtert die Verarbeitung bei der Herstellung der Filtertasche für den Bioreaktor.

Alternativ kann die Verstärkung der Membran für eine Verwendung als Filtertaschenwand durch das poröse Flächengebilde auch integral erfolgen, wie z.B. bei einer Zelluloseactetatmembran, die unverstärkt bei Kontakt mit Wasser eine Quellung von 0,7 - 1,3 % aufweist, die jedoch mit Polypropylenvlies integral verstärkt, in Wasser einen Quellungsgrad von nur noch 0,1 - 0,2 % aufweist. Oder wie bei einer Polyamidmembran (PA 6.6), die mit einem Polyestergewebe integral verstärkt ebenfalls eine Quellung von 0,1 - 0,2 % aufweist.

Bevorzugt besteht das poröse Flächengebilde und die Innenfläche der Wand des Bioreaktors, an welche die Filtertasche fixiert ist, aus Polymermaterialien, die aus der gleichen chemischen Stoffgruppe ausgewählt sind. Damit wird bei der Herstellung durch Verschmelzen der Polymermaterialien der Bioreaktorwand und des porösen Flächengebildes eine besonders dauerhafte und mechanisch stabile Verbindung erreicht.

Besonders bewährt hat sich ein poröses Flächengebilde, dessen Polymerfasern aus Kernmantelfasern bestehen, bei denen der Kern aus einem thermisch stabileren Polymer als das Mantelpolymer besteht. Durch die Verbindung des mikroporösen Filtermediums mit entsprechenden wenig quellenden oder schrumpfenden porösen Flächengebilden wird der Quellungs- und/oder Schrumpfungsgrad des Filtermediums weiter vermindert.

Eine besonders dauerhafte, fluiddichte Verbindung der mindestens einen Filtertasche mit der Innenfläche der Wand des Bioreaktors wird erreicht, wenn das Filtermedium in seinem peripheren Umfangsbereich an der Innenfläche der Wand des Bioreaktors durch Verschweißung fixiert ist, insbesondere wenn mindestens eine der Wände des Bioreaktors als eine flexible Wand ausgebildet ist.

Besonders vorteilhaft ist eine Filtertasche, welche flexibel ausgebildet ist. Dadurch ist es möglich, den kompletten Bioreaktor mit flexiblen Wänden nach einer Sterilisierung unter Inanspruchnahme eines geringen Volumens gebrauchsfertig verpackt an den Verbraucher zu versenden. Der erfindungsgemäße Bioreaktor ist hitze-, dampf-, strahlen- oder gassterilisierbar ausgeführt. Dadurch, dass die Filtertasche flexibel ist, nimmt sie die Form einer Schikane an, sobald der Bioreaktor mit flexiblen Wänden von einem äußere Schikanen ausformenden Stützbehälter aufgenommen ist. Das führt zu einer verbesserten Durchmischung des Kulturmediums und einer damit verbundenen erhöhten Leistungsfähigkeit des Bioreaktors.

In einer weiteren Ausführungsform der Erfindung besteht das Filtermedium für spezielle Anwendungen, bei denen gezielt Stoffe gewonnen werden sollen, aus Ultrafiltrationsmaterialien mit Ausschlussgrenzen (cut off) in dem Bereich zwischen etwa 500 bis etwa 1 Mio. Dalton.

Für den Fall, dass bei der Perfusion bestimmte Stoffe, beispielsweise toxische Stoffe oder Viren nicht in das Perfundat gelangen sollen, besteht das Filtermedium aus Membranadsorbern. Unter Membranadsorber werden Ionenaustauschermembranen, Adsorptionsmembranen oder aktive Membranen, insbesondere biologisch aktive Membranen verstanden. In einer weiteren bevorzugten Ausführung der Erfindung schließt die Filtertasche in ihrem Sammel- und/oder Verteilerraum einen Abstandshalter ein. Als konstruktiv günstig hat es sich erwiesen, wenn der Anschluss für das Zu- und/oder Abführen von Medien durch die Wand des Bioreaktors geführt ist.

Der erfindungsgemäße Bioreaktor verfügt in einer weiteren Ausführung über eine Mischvorrichtung mit der das Kulturmedium gemischt wird. Bevorzugt sind Mischvorrichtungen, die als Kipp-, Wipp-, Oszillations-, Vibrations-, Schüttel-, Begasungs-, Rühr-, Umpump- und/oder Pneumatikvorrichtungen ausgebildet sind.

Die Erfindung wird durch die nachstehenden Figuren und ein Ausführungsbeispiel näher erläutert.

Dabei zeigen
Figur 1 eine schematische Übersicht eines erfindungsgemäßen Bioreaktors mit einer Filtertasche und
Figur 2 einen detaillierten Aufbau einer Filtertasche.

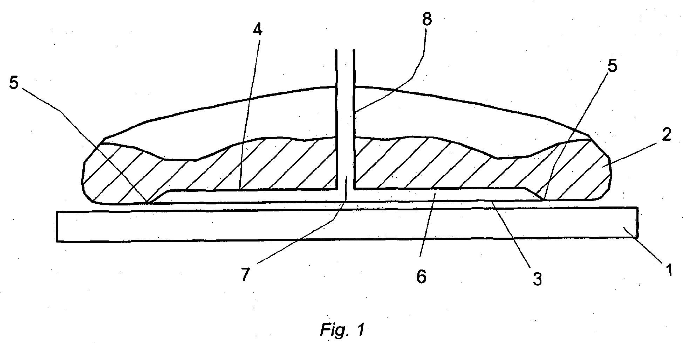

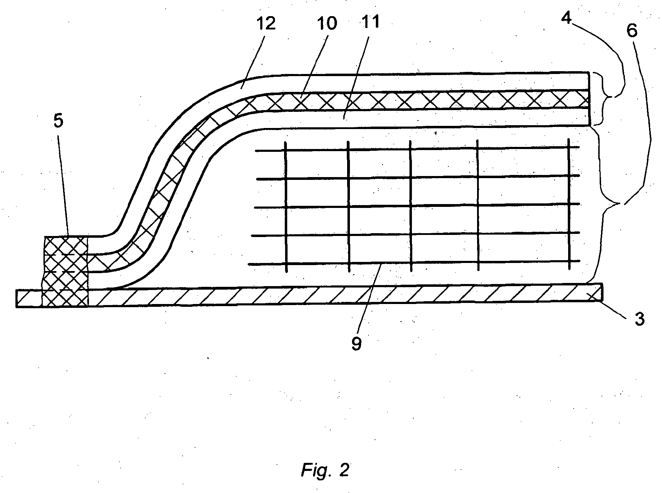

Gemäß Figur 1 liegt der Bioreaktor auf einer Mischvorrichtung 1, durch deren Bewegung das Kulturmedium 2 durchmischt wird. Auf der Innenfläche der unteren Wand 3 des Bioreaktors ist ein Filtermedium 4 in seiner Peripherie mit der Bioreaktorwand fixiert 5. Durch die Wand des Bioreaktors 3 und das Filtermedium 4 wird ein Sammel- /Verteilerraum 6 begrenzt, der an einer Stelle durch einen Anschluss 7 durchbrochen wird und über den mittels eines Schlauches 8 Medien dem Sammel- /Verteilerraum zu- oder abgeführt werden können.
Gemäß Figur 2 wird eine Filtertasche aus der Wand des Bioreaktors 3 und dem Filtermedium 4 gebildet. Im Sammel- und Verteilerraum 6 befindet sich ein Abstandshalter 9, der das Kollabieren des Sammel- / Verteilerraums verhindert. Das Filtermedium 4 ist in seiner Peripherie an die Wand des Bioreaktors 3 fixiert 5. Das Filtermedium 4 besteht aus einer Membran 10, die auf der dem Innenraum des Bioreaktors abgewandten Seite mit einem porösen Flächengebilde 11 verbunden ist. Als weitere Ausführungsform ist die Membran außerdem auch an der dem Innenraum des Bioreaktors zugewandten Seite mit einem porösen Flächengebilde 12 verbunden.

Ausführungsbeispiel:
Ein als Beutel mit integrierter Perfusionsmembran ausgeführter 10 Liter Bioreaktor Cultibag RM^{®} wird auf einer auf 37°C beheizten Wippplattform eingespannt und an eine BIOSTAT^{®}-Steuereinheit angeschlossen. Dazu ist die Oberseite des Beutels mit einer Vielzahl von Durchführungen versehen, die der Befüllung, dem Gasaustausch, dem Anschluss der Lichtleiter für an Schlauchenden lose in den Kulturraum eintauchende pH- und Sauerstoffsensoren, der Probennahme sowie der Ableitung des Perfundates dienen.
Der Bioreaktor ist mit einer auf seine Mittelwand aufgeschweißten Filtertasche ausgerüstet, deren bei Benetzung nicht quellendes Filtermedium vollständig aus einer hydrophilen Polyethersulfonmembran (Porengröße 1,2 µm) besteht, die einseitig mit einem stabilen Polypropylen/Polyethylen-Kernmantelvlies (100 g /m²) adhäsiv verbunden ist. Eine Schlaucholive (4 mm Innenradius) ist in das Filtermedium so eingeschweißt, dass im Kulturraum des Beutels eine Schlauchverbindung mit einer in der gegenüberliegenden Beutelwand vorgesehenen Doppelolive hergestellt werden kann, durch die eine Ableitung des Perfundates nach außen möglich ist.
Das verstärkte Filtermedium in den Abmessungen 25 x 40 cm ist mit der Vliesseite durch eine 5 mm breite Schweißnaht innen mit der dem Kulturmedium zugewandten Polyethylenschicht der dreischichtigen Innenfolie des Reaktorbeutels fluiddicht verbunden. Als Abstandshalter zwischen Innenfolie und Filtermedium ist ein auf 23 x 38 cm zugeschnittenes technisches Textil (Stärke 0,6 mm) in die Filtertasche eingelegt. Chinese-Hamster-Ovary-(CHO) -Zellen einer Vorkultur (3*10⁶ Zellen /ml) werden mit ProCHO5-Medium auf 2,5 Liter Zellsuspension der Zelldichte 5*10⁵ verdünnt und unter sterilen Bedingungen bei 37 °C in den 10 Liter Bioreaktor überführt. Mit 16 Wippbewegungen ("Rocking rate") pro Minute durchmischt startet die Zellreproduktion nach der Zugabe von Nährmedium unter einem 6 mbar Gaspolster, konstantem Sauerstoffgehalt und konstantem pH-Wert. Durch tägliche Probennahme wird die Zellvermehrung bis zur Zielkonzentration von 3*10⁶ /ml verfolgt. Nach Auffüllen auf 5 Liter Endvolumen und bei 20 Wippbewegungen pro Minute beginnt die Perfusion der Zellkultur bei einer Austauschrate von 3,5 ml /min (5 l /d) durch die integrierte Membran. Proben des Perfundates werden während der 7-tägigen Perfusion routinemäßig auf Ammonium, Lactat und den Zellgehalt überprüft.
Während des gesamten Kulturzeitraumes wird das Filtrationsmedium im Takt der Wippbewegung überspült und von den losen Einbauten des Bioreaktors touchiert. Eine dadurch hervorgerufene Beschädigung des Filtermediums bzw. ein Abplatzen oder Einreißen der Membranschicht an den Verbindungsflächen mit der Innenwand des Bioreaktors würde sofort eine hohe Zellkonzentration des Perfundates zur Folge haben.

Ergebnis: Während der Perfusion konnten keine Zellen im Perfundat gefunden werden. Das Filtermedium lag während der Kultur faltenfrei flach auf der Filtertasche. Zur Bestätigung der Unversehrtheit des hydrophilen Filtermediums und der Stabilität der Filtertasche wurde die Filtertasche einem "back pressure" Test unterzogen, bei dem die Filtertasche im flüssigkeitsgefüllten Bioreaktor aufgeblasen wird. Bis 90 mbar Rückseitendruck waren keine Luftblasen zu erkennen. Erst bei zunehmendem Druck traten vereinzelte Bläschenspuren in der Nähe des Siegelrandes (Bereich größter Dehnung) auf.

## Patentansprüche

1. Bioreaktor mit mindestens einer Filtertasche,
die an der Innenfläche einer Wand des Bioreaktors (3) fixiert ist und
in ihrem Inneren über einen Sammel- und/oder Verteilerraum (6) verfügt, der mit mindestens einem Anschluss (7) für ein Zu- und/oder Abführen von Medien kommunizierend verbunden ist,
wobei wenigstens eine Filtertaschenwand zumindest teilweise durch ein hydrophiles Filtermedium (4) gebildet ist,
**dadurch gekennzeichnet, dass**
das Filtermedium in Wasser einen Quellungs- und/oder Schrumpfungsgrad von höchstens 1 % aufweist.

2. Bioreaktor nach Anspruch 1, wobei die eine Wand des Bioreaktors (3) eine der Filtertaschenwände bildet.

3. Bioreaktor nach Anspruch 1, wobei die mindestens eine Filtertasche über Distanzhalter an der Innenseite der Wand des Bioreaktors (3) fixiert ist.

4. Bioreaktor nach einen der vorstehenden Ansprüche, wobei die wenigstens eine Filtertaschenwand zumindest teilweise durch ein hydrophobes Filtermedium (4) gebildet ist.

5. Bioreaktor nach Anspruch 4, wobei der Sammel- und/oder Verteilerraum (6) der mindestens einen Filtertasche fluiddicht unterteilt ist, derart, dass das hydrophile Filtermedium (4) an den Sammel- (6) und das hydrophobe Filtermedium (4) an den Verteilerraum (6) angrenzt.

6. Bioreaktor nach Anspruch 1, wobei das Filtermedium aus einer Membran (10) besteht, die auf mindestens der Seite, die dem Sammel- und/oder Verteilerraum (6) der Filtertasche zugewandt ist, mit einem porösen Flächengebilde aus thermoplastischen Polymerfasern (11) verbunden ist.

7. Bioreaktor nach Anspruch 6, wobei das poröse Flächengebilde (11) aus thermoplastischen Polymerfasern hydrophob ist und eine hydrophobe Barriere für das Kulturmedium darstellt, welche ab einer Druckdifferenz von 20 mbar überwindbar ist.

8. Bioreaktor nach einen der Ansprüche 6 bis 7, wobei die Membran auf beiden Seiten mit einem porösen Flächengebilde (11,12) aus thermoplastischen Polymerfasern verbunden ist, wobei das poröse Flächengebilde (12), dass nicht an den Sammel- und/oder Verteilerraum (6) der Filtertasche angrenzt, eine hydrophobe Barriere für das Kulturmedium darstellt, welche ab einer Druckdifferenz von 1 mbar überwindbar ist.

9. Bioreaktor nach einem der Ansprüche 6 bis 7, wobei das poröse Flächengebilde (11,12) und die Innenfläche der Wand des Bioreaktors, an welche die Filtertasche fixiert ist, aus Polymermaterialien bestehen, die aus der gleichen chemischen Stoffgruppe ausgewählt sind.

10. Bioreaktor nach den Ansprüchen 6 bis 7 und 9, wobei die Fixierung der Filtertasche mit der Innenfläche der Wand des Bioreaktors über das poröse Flächengebilde (11, 12) erfolgt ist.

11. Bioreaktor nach Anspruch 1, wobei mindestens eine Wand des Bioreaktors als eine flexible Wand ausgebildet ist.

12. Bioreaktor nach einem der vorstehenden Ansprüche, wobei die mindestens eine Filtertasche flexibel ausgebildet ist.

13. Bioreaktor nach den Ansprüchen 1 oder 4, wobei das hydrophile und/oder hydrophobe Filtermedium (4) mikroporös ist.

14. Bioreaktor nach den Ansprüchen 1, wobei das hydrophile Filtermedium (4) Ausschlussgrenzen in dem Bereich zwischen etwa 500 bis etwa 1 Mio. Dalton aufweist.

15. Bioreaktor nach Anspruch 1, wobei das hydrophile Filtermedium (4) ein Membranadsorber ist.

## Claims

1. Bioreactor with at least one filter pocket, which is fixed to the inner surface of a wall of the bioreactor (3) and has in its interior a collecting and/or distributing chamber (6) in communicating connection with at least one connection (7) for feed and/or discharge of media, wherein at least one filter pocket wall is formed at least partly by a hydrophilic filter medium (4), **characterised in that** the filter medium in water has a swelling and/or shrinkage factor of at most 1%.

2. Bioreactor according to claim 1, wherein such wall of the bioreactor (3) forms one of the filter pocket walls.

3. Bioreactor according to claim 1, wherein the at least one filter pocket is fixed to the inner side of the wall of the bioreactor (3) by way of a spacer.

4. Bioreactor according to any one of the preceding claims, wherein the at least one filter pocket wall is formed at least partly by a hydrophobic filter medium (4).

5. Bioreactor according to claim 4, wherein the collecting and/or distributing chamber (6) of the at least one filter pocket is fluid-tightly divided in such a way the hydrophilic filter medium (4) adjoins the collecting chamber (6) and the hydrophobic filter medium (4) adjoins the distributing chamber (6).

6. Bioreactor according to claim 1, wherein the filter medium consists of a membrane (10) which on at least one side which faces the collecting and/or distributing chamber (6) of the filter pocket is connected with a porous area body of thermoplastic polymer fibres (11).

7. Bioreactor according to claim 6, wherein the porous area body (11) of thermoplastic polymer fibres is hydrophobic and represents a hydrophobic barrier to the culture medium, which can be overcome from a pressure difference of 20 mbar.

8. Bioreactor according to one of claims 6 and 7, wherein the membrane is connected on both sides with the porous area body (11, 12) of thermoplastic polymer fibres, and wherein the porous area body (12), which does not adjoin the collecting and/or distributing chamber (6) of the filter pocket, represents a hydrophobic barrier for the culture medium, which can be overcome from a pressure difference of 1 mbar.

9. Bioreactor according to one of claims 6 and 7, wherein the porous area body (11, 12) and the inner surface of the wall of the bioreactor to which the filter pocket is fixed consist of polymer materials selected from the same chemical group of substances.

10. Bioreactor according to any one of claims 6 to 7 and 9, wherein the fixing of the filter pocket to the inner surface of the wall of the bioreactor is carried out by way of the porous area body (11, 12).

11. Bioreactor according to claim 1, wherein at least one wall of the bioreactor is constructed as a flexible wall.

12. Bioreactor according to any one of the preceding claims, wherein the at least one filter pocket is of flexible construction.

13. Bioreactor according to claim 1 or 4, wherein the hydrophilic and/or hydrophobic filter medium (4) is or are microporous.

14. Bioreactor according to claim 1, wherein the hydrophilic filter medium (4) has exclusion limits in the range between approximately 500 to approximately 1 million Daltons.

15. Bioreactor according to claim 1, wherein the hydrophilic filter medic medium (4) is a membrane adsorber.

## Revendications

1. Bioréacteur pourvu d'au moins une poche filtrante, laquelle est fixée contre la face intérieure d'une paroi du bioréacteur (3) et dont l'intérieur contient un compartiment de collecte et de distribution (6) relié de manière communiquante à au moins un raccord (7) pour l'amenée et/ou l'évacuation de fluides,
où au moins une paroi de poche filtrante est au moins partiellement formée par un milieu filtrant (4) hydrophile,
**caractérisé en ce que**
le milieu filtrant présente un degré de gonflement et/ou de rétraction dans l'eau de 1 % au maximum.

2. Bioréacteur selon la revendication 1, où la première paroi du bioréacteur (3) constitue une des parois de poche filtrante.

3. Bioréacteur selon la revendication 1, où ladite au moins une poche filtrante est fixée par des espaceurs contre la face intérieure de la paroi du bioréacteur (3).

4. Bioréacteur selon l'une des revendications précédentes, où ladite au moins une paroi de poche filtrante est au moins partiellement formée par un milieu filtrant (4) hydrophobe.

5. Bioréacteur selon la revendication 4, où le compartiment de collecte et de distribution (6) de ladite au moins une poche filtrante est subdivisé de manière étanche aux fluides, de telle manière que le milieu filtrant (4) hydrophile est adjacent au compartiment de collecte (6) et le milieu filtrant (4) hydrophobe au compartiment de distribution (6).

6. Bioréacteur selon la revendication 1, où le milieu filtrant est constitué d'une membrane (10), laquelle est raccordée, au moins sur la face opposée au compartiment de collecte et de distribution (6) de la poche filtrante, à une étoffe textile poreuse en fibres polymères thermoplastiques (11).

7. Bioréacteur selon la revendication 6, où l'étoffe textile poreuse (11) en fibres polymères thermoplastiques est hydrophobe et forme une barrière hydrophobe pour le milieu de culture, laquelle peut être traversée à partir d'un différentiel de pression de 20 mbar.

8. Bioréacteur selon l'une quelconque des revendications 6 à 7, où la membrane est raccordée sur ses deux faces à une étoffe textile poreuse (11, 12) en fibres polymères thermoplastiques, où l'étoffe textile poreuse (12) qui n'est pas adjacente au compartiment de collecte et de distribution (6) de la poche filtrante forme une barrière hydrophobe pour le milieu de culture, laquelle peut être traversée à partir d'un différentiel de pression de 1 mbar.

9. Bioréacteur selon l'une quelconque des revendications 6 à 7, où l'étoffe textile poreuse (11, 12) et la face intérieure de la paroi du bioréacteur contre laquelle la poche filtrante est fixée sont en matériaux polymères sélectionnés dans le même groupe de substances chimiques.

10. Bioréacteur selon les revendications 6, 7 et 9, où la fixation de la poche filtrante à la face intérieur de la paroi du bioréacteur est réalisée par l'étoffe textile poreuse (11, 12).

11. Bioréacteur selon la revendication 1, où au moins une paroi du bioréacteur est réalisée comme paroi flexible.

12. Bioréacteur selon l'une des revendications précédentes, où ladite au moins une poche filtrante est réalisée de manière flexible.

13. Bioréacteur selon les revendications 1 ou 4, où le milieu filtrant (4) hydrophile et/ou hydrophobe est microporeux.

14. Bioréacteur selon la revendication 1, où le milieu filtrant (4) hydrophile présente un seuil de coupure compris entre env. 500 et env. 1 million de Dalton.

15. Bioréacteur selon la revendication 1, où le milieu filtrant (4) hydrophile est un adsorbeur à membrane.
